# EUROPÄISCHE PATENTSCHRIFT

(11) **EP 2 597 453 B1**
(45) Veröffentlichungstag und Bekanntmachung des Hinweises auf die Patenterteilung: **17.08.2016**
(21) Anmeldenummer: 12182836.2
(22) Anmeldetag: 03.09.2012
(51) Int. Cl.: G01N 3/40, G01N 33/15, A61K 9/20, G01N 33/00

(54) **Belastungsmesseinrichtung sowie Verfahren zur mechanischen Belastungsprüfung**
Load measuring device and method for mechanical load testing
Dispositif de mesure de charge ainsi que procédé de test mécanique

(30) Priorität: 24.11.2011 DE 102011055701
(43) Veröffentlichungstag der Anmeldung: 29.05.2013
(73) Patentinhaber: SD Mechatronik GmbH, 83620 Feldkirchen-Westerham (DE)
(72) Erfinder: Duy, Sebastian, 83620 Feldkirchen (DE); Duy, Werner, 83620 Feldkirchen (DE); Korn, Benjamin, 83059 Kolbermoor (DE)
(74) Vertreter: 2s | ip Schramm Schneider Patentanwälte Rechtsanwälte

(56) Entgegenhaltungen:
- DE-A1-102009 008 288
- "Technology for Dental Materials Research", , 31. Dezember 2007 (2007-12-31), XP055118329, Gefunden im Internet: URL:http://www.sdm-gmbh.de/fileadmin/defau lt/PDF/produktkatalog.pdf [gefunden am 2014-05-15]
- Enrico Conserva ET AL: "Robotic chewing simulator for dental materials testing on a sensor-equipped implant setup", Int J Prosthodont, 1. Januar 2008 (2008-01-01), Seiten 501-508, XP055118523, Gefunden im Internet: URL:http://www.researchgate.net/publicatio n/23796164_Robotic_chewing_simulator_for_d ental_materials_testing_on_a_sensor-equipp ed_implant_setup/file/79e4151116ac4e483e.p df [gefunden am 2014-05-16]
- Kanal von SDMechatronik-: "Chewing simulator with Thermocycling", , 7. Juni 2011 (2011-06-07), XP054975407, Gefunden im Internet: URL:https://www.youtube.com/watch?v=Nz-Npn KSD2w [gefunden am 2014-05-19]

## Beschreibung

Die Erfindung betrifft eine Belastungsmesseinrichtung, insbesondere zum Messen physikalischer Parameter einer Probe bei einer mechanischen Krafteinwirkung auf die Probe, sowie ein Verfahren zum Messen physikalischer Parameter einer Probe bei einer mechanischen Krafteinwirkung auf die Probe, mit einer erfindungsgemäßen Belastungsmesseinrichtung. Insbesondere betrifft die Erfindung eine Belastungsmesseinrichtung, mit der mechanische Eigenschaften einer Probe, etwa eine Tablette, bei Belastung der Probe durch eine Kaubewegung untersucht werden können, wobei die Belastungsmesseinrichtung ausgestaltet ist Kaubewegungen zu simulieren.

### Hintergrund der Erfindung und Stand der Technik

Bei der Entwicklung bzw. Herstellung von Tabletten, Dragees, Pastillen oder sonstigen einzeldosierten festen Arzneimitteln ist es wichtig, die mechanischen Eigenschaften der Produkte, im Folgenden Probe genannt, bei einer mechanischen Krafteinwirkung auf das Produkt bzw. auf die Probe zu kennen. Wünschenswert ist es hierbei, wenn die mechanischen Eigenschaften der Probe bei einer Belastung durch eine Kaubewegung untersucht werden können. Im Rahmen der Produktentwicklung ist es von besonderem Interesse, Informationen über das Verhalten von Tabletten im Mund, insbesondere während einer Kaubewegung, zu erlangen. Beispielsweise sollen Informationen über das Bruchverhalten der Probe bei Belastung mit einer definierten Kraft, die Anzahl der Kaubewegungen, die notwendig ist, um einen Bruch der Probe herbeizuführen oder über das Lösungsverhalten der Probe in einem wässrigen Milieu bei einer vorbestimmten Temperatur erlangt werden.

Vorrichtungen zum Prüfen der Bruchfestigkeit von Tabletten sind aus dem Stand der Technik bekannt. So ist beispielsweise aus der DE 10 2006 004 215 B4 ein Bruchfestigkeitstester zur Bruchfestigkeitsprüfung von Tabletten bekannt. Der Bruchfestigkeitstester umfasst ein Hauptgehäuse und einen daran befestigten Positionierer, welcher zur Aufnahme der Tablette vorgesehen ist. Der Positionierer wird seitlich von einer Anschlagfläche begrenzt. Das Hauptgehäuse umfasst einen ausfahrbaren Bruchbacken, der gegen die als ortsfester Bruchbacken dienende Anschlagfläche des Positionierers bewegt werden kann. Der bewegliche Bruchbacken wird während eines Testzyklus in Richtung des ortsfesten Bruchbackens verfahren, sodass eine Kraft auf die zwischen den beiden Bruchbacken angeordnete Tablette ausgeübt wird. Der bewegbare Bruchbacken ist mit einer Messeinrichtung verbunden, mit welcher die auf die eingespannte Tablette ausgeübte Kraft gemessen und aufgezeichnet werden kann. Der bewegliche Bruchbacken ist mit einem Schrittmotor gekoppelt, sodass bei jedem Schritt des Schrittmotors die auf die Tablette wirkende Kraft ansteigt, bis die Tablette schließlich zerbricht. Die hierfür aufgewendete Kraft dient als Maß für die Bruchfestigkeit der Tablette.

Nachteilig bei dem aus dem Stand der Technik bekannten Bruchfestigkeitstester ist, dass lediglich die Belastung in eine Richtung gemessen werden kann. Ein weiterer Nachteil besteht darin, dass eine Eindringtiefe des Bruchbackens in die Tablette nicht gemessen werden kann, weil die Anschlagflächen der Bruchbacken eine im Wesentlichen ebene Oberfläche aufweisen, sodass die Bruchbacken schon gar nicht in die Tablette bzw. Probe eindringen können. Ferner ist es nachteilig, dass das Bruchverhalten der Probe in einem wässrigen Milieu nicht geprüft werden kann. Das Prüfen des Bruchverhaltens der Probe in einem wässrigen Milieu ist aber wichtig, weil sich die mechanischen Eigenschaften der Probe in einem wässrigen Milieu verändern können und somit auch zu einem veränderten Bruchverhalten führten können.

Aus der Internetveröffentlichung "Technology for Dental Material Research, Issue 2007, SD Mechatronik GmbH, Deutschland" ist eine Belastungsmesseinrichtung bekannt, die eine Probenkammer zur Aufnahme der Probe und einen Stab aufweist, wobei die Längsachse des Stabes senkrecht zur Probe in der Probenkammer steht, wobei der Stab entlang seiner Längsachse bewegbar ist und wobei die Probenkammer entlang einer Achse horizontal verfahrbar ist.

Aus der DE 10 2009 008 288 A1 ist eine Vorrichtung zur Härteprüfung von Werkstücken bekannt. Die weist einen Eindringkörper auf, der vertikal beweglich gelagert ist. Ein Haltetisch, an dem das Werkstück angeordnet ist, ist in horizontaler Richtung bewegbar.

Aus der Veröffentlichung "Enrico Conserva et al.: Robotic chewing simulator for dental materials testing on a sensor-equipped implant setup; The International Journal of Prosthodontics; Vol. 21, Number 6, 2008" ist ein Kausimulator bekannt, der eine bewegliche Plattform zur Aufnahme einer Probe aufweist. An der Plattform sind sechs Hydraulikarme angeordnet, wobei durch eine Änderung der Länge der Hydraulikarme die Plattform um eine bestimmte Winkel rotiert und translatorisch bewegt werden kann.

### Aufgabe der Erfindung

Der Erfindung liegt daher die Aufgabe zugrunde, eine Belastungsmesseinrichtung, insbesondere zum Messen physikalischer Parameter einer Probe bei einer mechanischen Krafteinwirkung auf die Probe bereitzustellen, welche die aus dem Stand der Technik bekannten Nachteile zumindest teilweise vermeidet und welche es ermöglicht, zusätzlich zu der auf die Probe wirkende Kraft auch die Eindringtiefe eines Belastungskörpers in die Probe sowohl bei trockener Umgebung als auch in einem wässrigen Milieu zu ermitteln. Ferner soll es möglich sein, unterschiedliche Belastungsszenarien zu vermessen, ohne hierbei den Belastungskörper wechseln zu müssen.

### Erfindungsgemäße Lösung

Diese Aufgabe wird erfindungsgemäß durch eine Belastungsmesseinrichtung, insbesondere zum Messen physikalischer Parameter einer Probe bei einer mechanischen Krafteinwirkung auf die Probe, sowie ein Verfahren zum Messen physikalischer Parameter einer Probe bei einer mechanischen Krafteinwirkung auf die Probe nach den unabhängigen Ansprüchen gelöst. Vorteilhafte Ausgestaltungen und Weiterbildungen der Erfindung sind in den jeweiligen abhängigen Ansprüchen angegeben.

Bereitgestellt wird demnach eine Belastungsmesseinrichtung, insbesondere zum Messen physikalischer Parameter einer Probe bei einer mechanischen Krafteinwirkung auf die Probe, aufweisend
- eine Probenkammer zur Aufnahme der Probe, wobei die Probenkammer entlang einer ersten Achse verfahrbar ist, und
- einen Stab, welcher entlang einer zweiten Achse relativ zur Probenkammer verfahrbar ist, wobei die zweite Achse im Wesentlichen senkrecht zur ersten Achse steht, und wobei an dem der Probenkammer zugewandten Ende des Stabes ein Belastungskörper angeordnet ist,
wobei der Stab drehbar um seine Lenksachse gelagert ist.

Dadurch können mit nicht-rotationssymmetrischen Belastungskörpern unterschiedliche Belastungsszenarien simuliert werden, in dem der Stab um verschiedene Winkel gedreht wird, ohne den Belastungskörper wechseln zu müssen.

Der Stab kann, über zumindest ein Axiallager, mit einer Trägerplatte zur Aufnahme von Gewichten gekoppelt sein.

Zum Verfahren des Stabes entlang der zweiten Achse kann eine erste Antriebseinheit vorgesehen sein, welche, vorzugsweise über eine erste Vorschubeinrichtung, mit einer entlang der zweiten Achse verfahrbaren Trägereinrichtung gekoppelt ist, wobei die Trägereinrichtung derart relativ zur Trägerplatte angeordnet ist, dass ein Verfahren der Trägereinrichtung ein Verfahren der Trägerplatte entlang der zweiten Achse bewirkt.
Die Belastungsmesseinrichtung kann ferner eine dritte Antriebseinheit aufweisen, welche, vorzugsweise über Koppelmittel, mit dem Stab gekoppelt ist, um den Stab um dessen Lenksachse zu drehen.
Die Koppelmittel können eine Zahnradkombination oder Zahnriemenkoppelung und eine Hülse umfassen, wobei die Zahnradkombination oder die Zahnriemenkoppelung mit der dritten Antriebseinheit und mit der Hülse gekoppelt ist, wobei die Hülse den Stab zumindest teilweise umgibt, und wobei die Hülse an der Innenwandung einen Stift aufweist, welcher in ein parallel zur Längsache verlaufendes Langloch am Stab eingreift.
In der Hülse kann zumindest ein Linearlager angeordnet sein, in welchem der Stab im Wesentlichen spielfrei entlang seiner Längsachse geführt ist.
Die dritte Antriebseinheit und die Koppelmittel können an der Trägereinrichtung angeordnet sein, wobei die Trägereinrichtung einen Durchbruch aufweist, in welchem die Hülse in zumindest einem Radiallager drehbar gelagert ist.

Die Probenkammer kann auf einem Probentisch angeordnet sein, welcher mit einer zweiten Antriebseinheit, vorzugsweise über eine zweite Vorschubeinrichtung, gekoppelt ist.

Zwischen dem Probentisch und der Probenkammer kann ein Kraftsensor, vorzugsweise ein Mehrkomponenten-Kraftsensor, angeordnet sein. Vorteilhaft ist es, wenn ein Wegsensor, vorzugsweise ein induktiver Wegsensor zum Messen des vertikalen Verfahrweges des Stabes vorgesehen ist.
In der Probenkammer können eine Heiz-/Kühleinrichtung, eine Umwälzpumpe und eine Platte zur Aufnahme einer Probenhalterung angeordnet sein.

Die Probenkammer kann mehrteilig ausgestaltet sein, wobei ein Unterteil und ein Oberteil der Probenkammer zusammen eine erste Kammer bilden, in der die Umwälzpumpe angeordnet ist, wobei die Platte an der Oberseite des Oberteils angeordnet ist und mit dem Oberteil eine fluiddichte zweite Kammer bildet, in der die Heiz-/Kühleirichtung angeordnet ist, wobei die Heiz-/Kühleinrichtung vorzugsweise thermisch mit der Platte gekoppelt ist, wobei an der Oberseite des Oberteils ein offener Zylinder angeordnet ist, dessen Grundfläche von der Platte fluiddicht verschlossen wird, und wobei die erste Kammer über zumindest einen Kanal mit dem Innenraum des Zylinders verbunden ist.

Die erste Vorschubeinrichtung und / oder die zweite Vorschubeinrichtung können einen Kugelgewindetrieb mit einer Kugelgewindespindel und einer Kugelgewindemutter umfassen. Die erste Antriebseinheit und / oder die zweite Antriebseinheit können einen Servomotor umfassen. Die dritte Antriebseinheit kann einen Schrittmotor umfassen.
Bereit gestellt wird ferner ein Verfahren zum Messen physikalischer Parameter einer Probe bei einer mechanischen Krafteinwirkung auf die Probe, mit einer erfindungsgemäßen Belastungsmesseinrichtung, wobei das Verfahren zumindest umfasst:
- Anordnen der Probe in einer Probenkammer,
- Festlegen von Versuchsparametern der Belastungsmesseinrichtung, wobei die Versuchparameter zumindest eines aus Drehwinkel des Stabes, Winkelgeschwindigkeit des Stabes, und eine Kombination hiervon umfassen,
- Festlegen von Abbruchkriterien, wobei die Abbruchkriterien zumindest eines aus maximaler Eindringtiefe des Belastungskörpers in die Probe, maximaler Kraft in Y- und/oder Z-Richtung, maximaler Anzahl von Hubbewegungen, maximaler Drehwinkel des Stabes, und eine Kombination hiervon umfassen,
- Aufzeichnen von Messwerten, insbesondere Kraft/Zeitdiagramm in Y- und/oder Z-Richtung und Eindringtiefe.

Die Versuchsparameter können in einer Ausgestaltung der Erfindung weiter umfassen: Verfahrweg des Stabes, Verfahrweg der Probenkammer, Verfahrgeschwindigkeit des Stabes, Verfahrgeschwindigkeit der Probenkammer, und Anzahl von Hubbewegungen des Stabes entlang der zweiten Achse.

Das Verfahren kann des Weiteren umfassen:
- Füllen der Probenkammer mit einem Fluid, und
- Festlegen der Temperatur des Fluids als Versuchsparameter.

In einer Ausgestaltung der Erfindung kann das Festlegen der Versuchsparameter und / oder der Abbruchkriterien ein Auslesen vorbestimmter Werte aus einer Speichereinrichtung umfasst.

### Kurzbeschreibung der Figuren

Weitere Einzelheiten und Merkmale der Erfindung sowie konkrete, insbesondere vorteilhafte Ausführungsbeispiele der Erfindung ergeben sich aus der nachfolgenden Beschreibung in Verbindung mit der Zeichnung. Es zeigt:
- Fig. 1: eine perspektivische Ansicht einer erfindungsgemäßen Belastungsmesseinrichtung;
- Fig. 2: eine Seitenansicht einer erfindungsgemäßen Belastungsmesseinrichtung;
- Fig. 3: einen Schnitt durch eine erfindungsgemäße Belastungsmesseinrichtung in einer perspektivischen Ansicht;
- Fig. 4: einen Schnitt durch eine erfindungsgemäße Belastungsmesseinrichtung;
- Fig. 5: eine Detailansicht der Drehachse des Schnittes gemäß Fig. 4;
- Fig. 6: eine Detailansicht der Probenkammer gemäß dem Schnitt nach Fig. 4; und
- Fig. 7: einen Schnitt durch die Probenkammer in einer perspektivischen Ansicht.

### Detaillierte Beschreibung der Erfindung

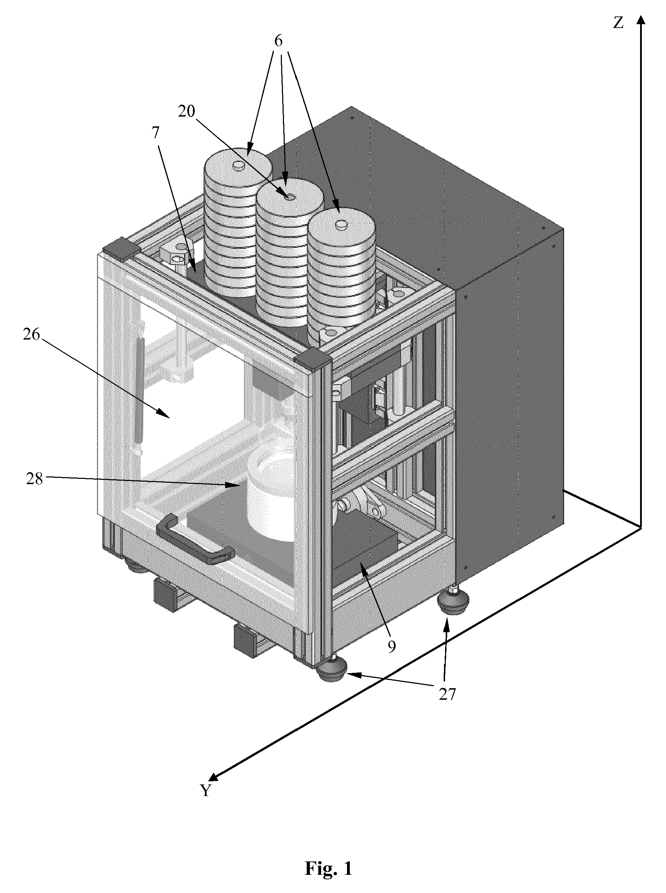

**Fig. 1** zeigt eine erfindungsgemäße Belastungsmesseinrichtung in einer perspektivischen Ansicht.

Die Belastungsmesseinrichtung umfasst ein Gehäuse, in welchem sämtliche Komponenten für die Belastungsmessung sowie sämtliche Antriebseinheiten angeordnet sind. Das Gehäuse weist an seiner Vorderseite eine Tür bzw. eine Klappe 26 auf, um Zugang zur Probenkammer 28 und/oder zum Belastungskörper im Inneren des Gehäuses zu erhalten.

Die Probenkammer 28 ist auf einem verfahrbaren Probentisch 9 angeordnet, welcher gemäß der vorliegenden Erfindung entlang der Y-Achse verfahrbar ist. In einer weiteren Ausgestaltung der Erfindung kann der Probentisch 9 auch entlang der X-Achse verfahren werden.

An der Oberseite des Gehäuses ragen mehrere Stäbe heraus, an welchen Gewichte 6 angeordnet werden können, indem die Gewichte 6 auf die Stäbe aufgesteckt werden. Die Gewichte 6 liegen auf einer Trägerplatte 7 auf, welche entlang der Z-Achse bewegbar ist.

An der Unterseite des Gehäuses sind Füße 27 vorgesehen. In einer bevorzugten Ausführungsform der Erfindung können die Füße 27 als schwingungsdämpfende Füße ausgestaltet sein.

Die Belastungsmesseinrichtung ist derart ausgestaltet, dass sie Bewegungen entlang der Y-Achse (durch den Probentisch 9) und entlang der Z-Achse (durch den Stab 20 bzw. durch die an dem Stab 20 befestigten Trägerplatte 7) durchführen kann. Die Bewegungen werden vorzugsweise jeweils mittels einer Antriebseinheit bewirkt. Ferner ist erfindungsgemäß der Stab 20 (in Fig. 1 der mittlere der drei Stäbe, an denen die Gewichte 6 angeordnet sind) um seine Längsachse drehbar gelagert.

Die Verfahrwege entlang der jeweiligen Achse, der Drehwinkel sowie die Geschwindigkeiten für einen Messzyklus können von dem Anwender vorgegeben werden. Die Kraft, mit der eine Probe belastet werden soll, wird durch die Anzahl der auf der Trägerplatte 7 angeordneten Gewichte 6 festgelegt. Sobald der - in Fig. 1 nicht sichtbare - Belastungskörper 5 die Oberfläche der Probe berührt, beginnt das aufgelegte Gewicht auf die Probe zu wirken.

Die Probenkammer 28 kann mit einem Fluid, etwa Wasser, gefüllt werden, sodass sich die Probe in der Probenkammer 28 teilweise oder vollständig im Fluid befindet. In der Probenkammer 28 ist eine Temperiereinrichtung vorgesehen, um das Fluid in der Probenkammer auf eine vorbestimmte Temperatur zu erwärmen.

Für das Ermitteln der Bewegungen entlang der jeweiligen Achse, der Drehbewegung des Stabes, der auf die Probe einwirkenden Kräfte sowie der Temperatur des Fluids in der Probenkammer sind mehrere Messeinrichtungen vorgesehen:
- ein Kraftsensor, um die Kräfte bei der Belastung der Probe in mehrere Richtungen zu messen, vorzugsweise ein Mehrkomponenten-Kraftsensor,
- ein Wegsensor, um die Eindringtiefe eines Belastungskörpers in die Probe zu messen, und/oder
- ein Temperatursensor, um die Temperatur des Fluids in der Probenkammer 28 zu messen.

Die Sensoren sind mit einer Steuer- bzw. Regeleinrichtung gekoppelt. Vorzugsweise erfolgt die Steuerung der Belastungsmesseinrichtung und die Aufnahme der Messwerte der Sensoren durch eine integrierte Datenverarbeitungsanlage (etwa ein Computer oder ein Mikrocontroller), welche mit einer Speichereinrichtung zum Speichern der aufgenommenen Messwerte gekoppelt ist. In der Speichereinrichtung können auch vorbestimmte Versuchsparameter für die Durchführung einer Messung gespeichert werden. Solche Versuchsparameter können beispielsweise vordefinierte Verfahrwege, vordefinierte Verfahrgeschwindigkeiten und/oder Beschleunigungen, Drehwinkel und/oder Winkelgeschwindigkeit, oder die Anzahl der durchzuführenden Kauzyklen umfassen.

Zusätzlich zu den Versuchsparametern können auch Abbruchkriterien für einen Versuch gespeichert werden. Beispielsweise können die Abbruchkriterien eine maximal zulässige Eindringtiefe des Belastungskörpers in die Probe, eine maximale auf die Probe einwirkende Kraft in X-, Y- oder Z-Richtung, ein Erreichen eines vorgegebenen Drehwinkels oder dergleichen umfassen.

Für die Auswertung der aufgenommenen Messwerte kann die Datenverarbeitungseinrichtung ausgestaltet sein, ein Kraft-/Zeitdiagramm für die drei Achsen zu erzeugen und zu speichern, die Eindringtiefe zu speichern, die maximale und/oder minimale Kraft in allen drei Achsen zu ermitteln und/oder zu speichern, sowie die maximale und/oder minimale Eindringtiefe zu ermitteln und/oder zu speichern. Eine Report-Funktion der Datenverarbeitungseinrichtung ermöglicht die Ausgabe der Messwerte in übersichtlicher Form.

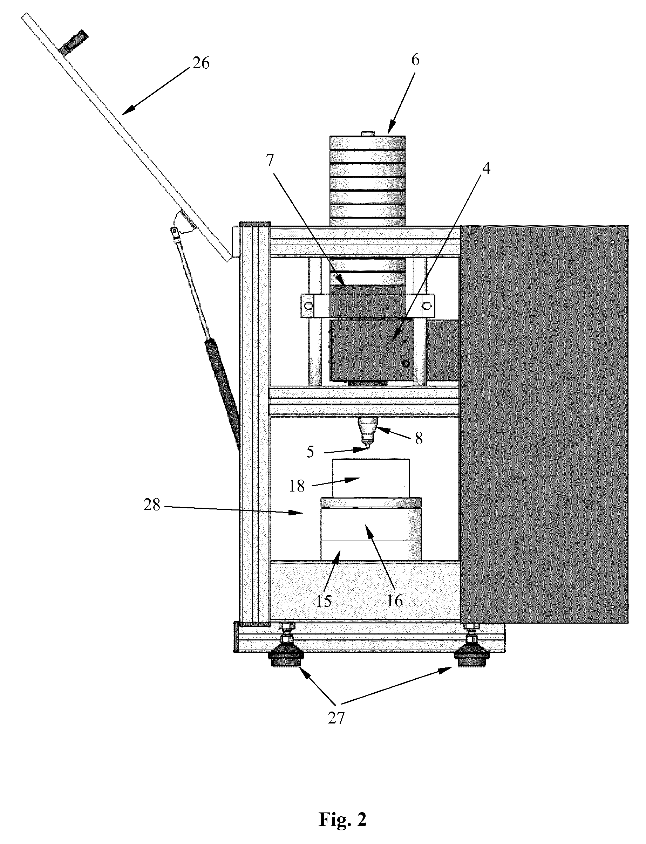

**Fig. 2** zeigt eine Seitenansicht der in Fig. 1 gezeigten Belastungsmesseinrichtung mit einer nach oben geöffneten Klappe 26. Selbstverständlich kann anstelle einer nach oben klappbaren Klappe 26 auch eine zur Seite schwenkbare Tür bzw. Klappe vorgesehen sein.

Erkennbar sind hier die auf der Trägerplatte 7 abgelegten Gewichte 6, sowie eine Trägereinrichtung 4, auf der die Trägerplatte 7 aufliegt. Die Trägereinrichtung 4 kann nach oben bzw. nach unten verfahren werden, sodass die auf der Trägereinrichtung aufliegende Trägerplatte 7 ebenfalls nach oben bzw. nach unten verfahren wird.

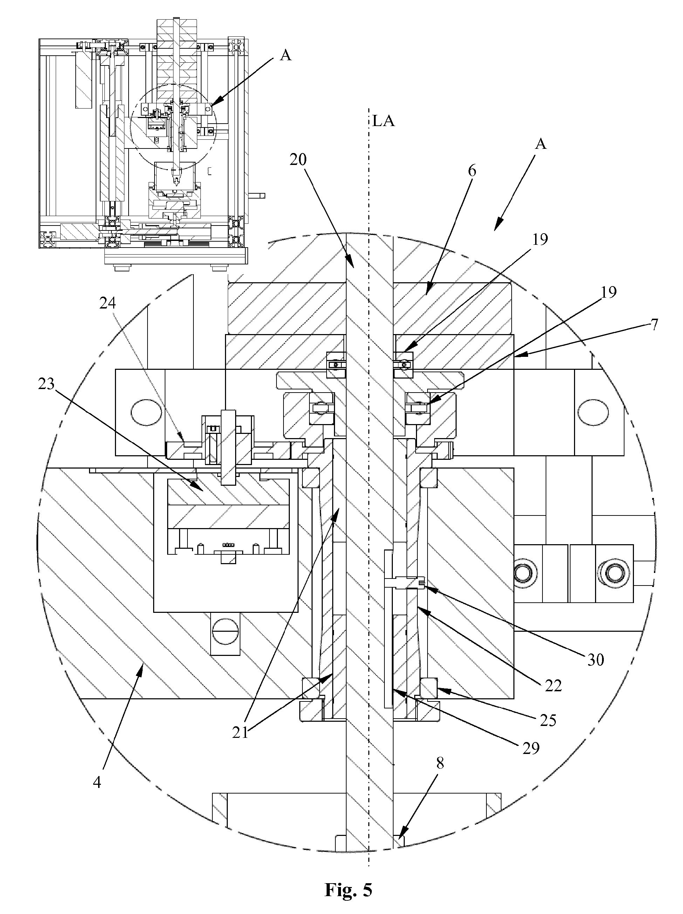

Ferner ist das untere Ende des mittleren Stabes 20 sichtbar. An der Unterseite des mittleren Stabes 20 ist ein Verbindungselement 8 zur Aufnahme eines Belastungskörpers 5 vorgesehen. Der mittlere Stab ist mit der Trägerplatte 7 (vgl. Fig. 5) gekoppelt, sodass auch der Belastungskörper 5 bei einem Verfahren der Trägereinrichtung 4 angehoben bzw. abgesenkt wird. Sobald der Belastungskörper 5 die in der Probenaufnahme 18 angeordnete Probe berührt, kann die Trägereinrichtung 4 noch weiter abgesenkt werden, sodass das vollständige Gewicht der auf der Trägerplatte 7 angeordneten Gewichte 6 auf die Probe wirken kann.

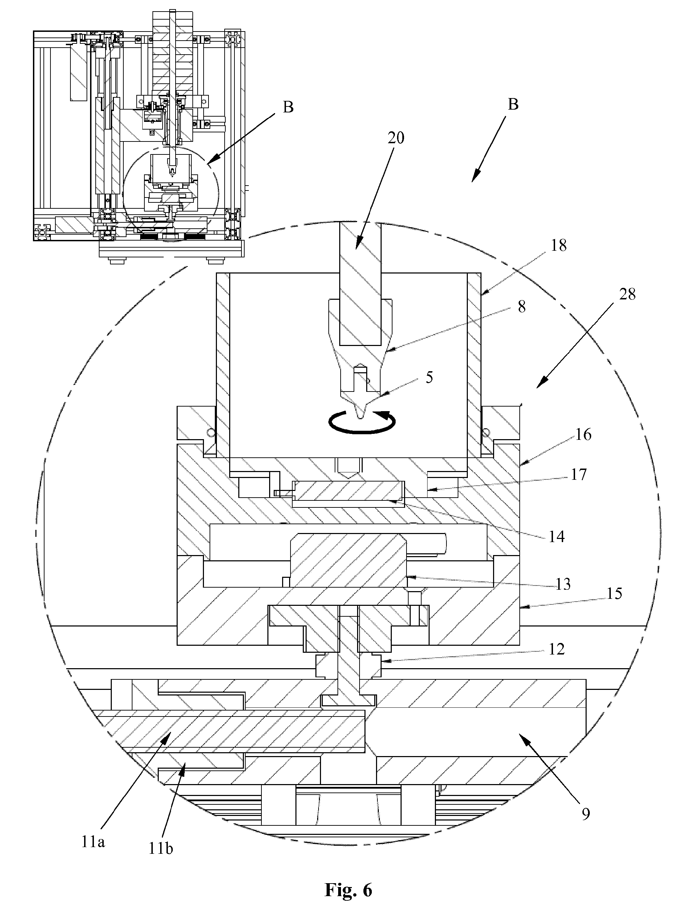

Die Probenkammer 28 weist ein Unterteil 15 und ein Oberteil 16 sowie einen an dem Oberteil angeordneten Zylinder 18 auf. Eine detaillierte Beschreibung der Probenkammer 28 erfolgt mit Bezug auf Fig. 6.

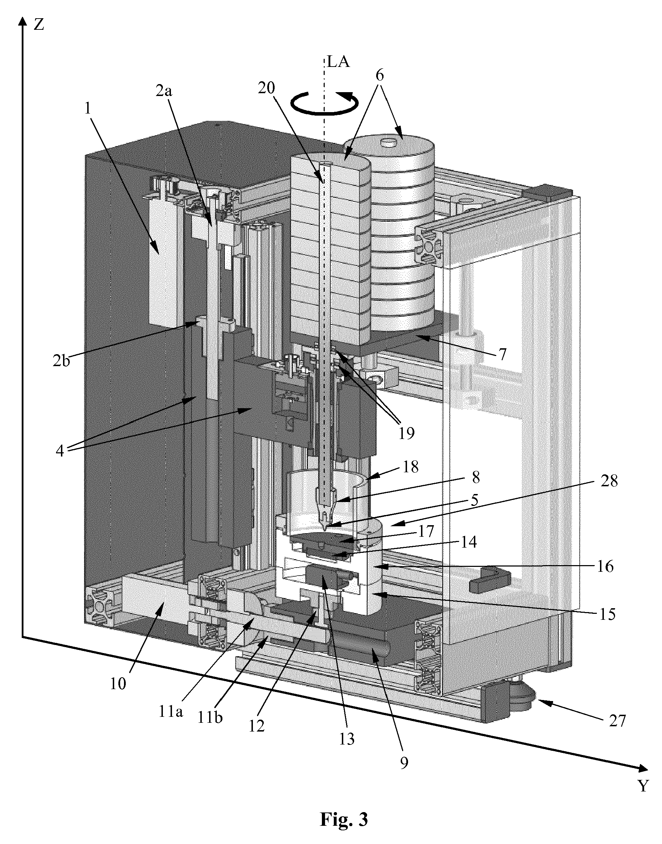

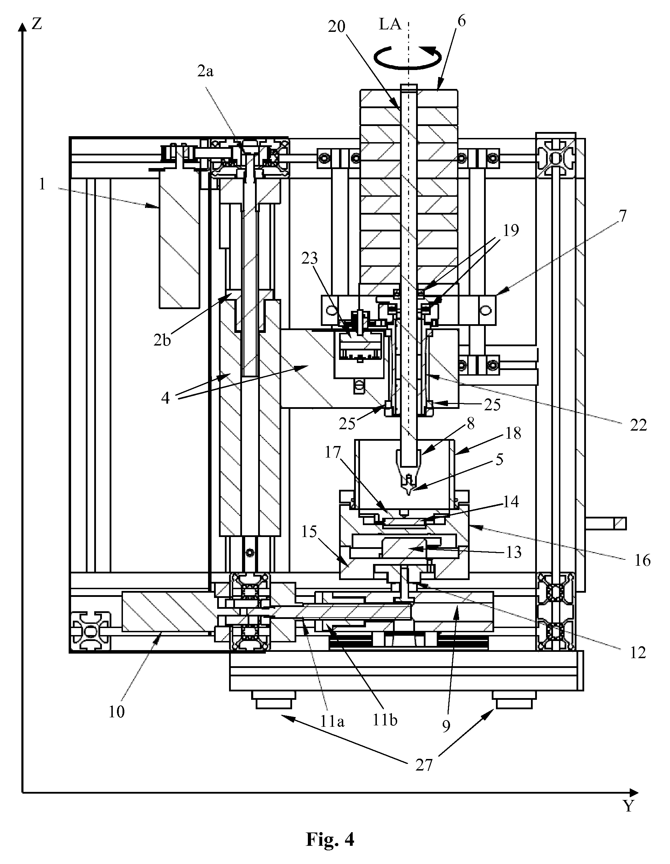

**Fig. 3** und **Fig. 4** zeigen jeweils einen Schnitt durch eine erfindungsgemäße Belastungseinrichtung.

An dem Stab 20 (mittlerer, der in Fig. 1 gezeigten drei Stäbe) sind Gewichte 6 angeordnet, welche auf der Trägerplatte 7 aufliegen. Die Trägerplatte 7 liegt im Wesentlichen auf der Trägereinrichtung 4 auf, welche mit einer ersten Vorschubeinrichtung 2a, 2b angehoben bzw. abgesenkt werden kann.

Die erste Vorschubeinrichtung 2a, 2b kann ein Kugelgewindetrieb sein, wobei die Kugelgewindemutter 2b mit der Trägereinrichtung 4 fest verbunden ist. Durch Drehen der Kugelgewindespindel 2a bewegt sich die Kugelgewindemutter 2b auf-bzw. abwärts, was zu einer Hubbewegung der mit der Kugelgewindemutter 2b verbundenen Trägereinrichtung 4 führt. Der Antrieb der Kugelgewindespindel 2a kann über eine erste Antriebseinheit 1 erfolgen, welche ein Servomotor sein kann und über einen Zahnriemen mit der Kugelumlaufspindel 2a gekoppelt ist.

Ist die Trägereinrichtung 4 so weit abgesenkt, dass der Belastungskörper 5 an dem unteren Ende des Stabes 20 auf der in der Probenkammer 28 angeordneten Probe aufliegt, kann die Trägereinrichtung 4 weiter abgesenkt werden, sodass sich die Trägereinrichtung 4 von der Trägerplatte 7 löst, sodass letztlich das gesamte auf der Trägerplatte 7 angeordnete Gewicht auf die Probe wirkt.

Der Belastungskörper 5 ist über ein Verbindungselement 8 an dem Stab 20 angeordnet, sodass sowohl das Verbindungselement 8 als auch der Belastungskörper 5 besonders einfach und schnell ausgewechselt werden können. Dadurch können Belastungskörper 5 unterschiedlicher Geometrie eingesetzt werden, um etwa verschiedene Belastungsszenarien zu simulieren.

Die Kraft des auf die Probe wirkenden Belastungskörpers 5 wird durch die Gewichte 6 bestimmt. Als Gewichte 6 können beispielsweise Stahlscheiben mit einem Gewicht von jeweils 1 kg vorgesehen sein. Selbstverständlich können auch Scheiben unterschiedlichen Gewichtes auf der Trägerplatte 7 angeordnet werden.

Im unteren Bereich der Belastungsmesseinrichtung ist ein Probentisch 9 vorgesehen, welcher zumindest entlang der Y-Achse verfahrbar ist. In einer hier nicht gezeigten Ausgestaltung der Belastungseinrichtung kann der Probentisch 9 auch entlang der X-Achse verfahrbar sein. Der Probentisch 9 ist über eine zweite Vorschubeinrichtung 11a, 11b, welche als Kugelgewindetrieb ausgestaltet sein kann, mit einer zweiten Antriebseinheit 10, welche als Servomotor ausgestaltet sein kann, gekoppelt. Die Kugelgewindemutter 11b des zweiten Kugelgewindetriebes ist fest mit dem Probentisch 9 verbunden, sodass eine Drehung der Kugelgewindespindel 11a eine Bewegung des Probentisches 9 entlang der Y-Achse bewirkt.

Auf dem Probentisch 9 ist die Probenkammer 28 angeordnet, wobei zwischen der Probenkammer 28 und dem Probentisch 9 ein Kraftsensor 12 angeordnet ist. Der Kraftsensor 12 ist fest mit dem Probentisch 9 verbunden. Dadurch wird erreicht, dass alle Kräfte, die auf die Probe einwirken, gemessen werden können. Vorzugsweise wird als Kraftsensor ein Mehrkomponenten-Kraftsensor vorgesehen, der Kräfte in allen drei Richtungen X/Y/Z messen kann.

Die auf dem Kraftsensor angeordnete Probenkammer 28 ist mehrteilig ausgestaltet und weist eine Umwälzpumpe 13, eine Heiz-/Kühleinrichtung 14 sowie eine vorzugsweise wärmeleitende Platte 17 auf. Der genaue Aufbau und Funktionsweise der Probenkammer 28 wird mit Bezug auf Fig. 6 näher beschrieben.

**Fig. 5** zeigt eine Detailansicht des Stabes 20 in einer Schnittansicht.

Am unteren Ende des Stabes 20, welcher vorzugsweise zylinderförmig ausgestaltet ist, ist ein Verbindungselement 8 zur Aufnahme des Belastungskörpers 5 angeordnet. Sowohl das Verbindungselement 8 als auch der Belastungskörper 5 können von dem Stab 20 bzw. von dem Verbindungselement 8 gelöst werden. Dadurch ist ein einfacher Austausch des Belastungskörpers 5 möglich.

Der Stab 20 (mittlere Stab der in Fig. 1 gezeigten drei Stäbe) ist entlang seiner Längsachse LA (d.h. entlang der Z-Achse) bewegbar. Ferner ist der Stab 20 drehbar um seine Längsachse LA gelagert.

Mit der vertikalen Bewegung des Stabes 20 kann ein Eindringen des Belastungskörpers 5 in die Probe simuliert werden. Durch horizontales Bewegen des Probentisches 9 kann die Position an der der Belastungskörper 5 eindringt festgelegt werden. Durch die Drehbarkeit des Stabes 20 um seine Längsachse LA können zudem verschiedene Eindringwinkel des Belastungskörpers 5 in die Probe eingestellt werden, insbesondere dann wenn es sich bei dem Belastungskörper 5 nicht um einen rotationssymmetrischen Belastungskörper handelt.

Durch die vertikale Bewegung des Stabes 20, die horizontale Bewegung des Probentisches 9 und die Drehbarkeit des Stabes 20 um seine Längsachse LA kann eine besonders realistische Simulation eines Kauvorganges gewährleistet werden, weil eine Tablette bzw. ein Dragee im Mund durch die Zunge bzw. durch den Kauvorgang selbst an verschiedene Stellen des Kiefers gebracht werden kann. Dabei ändert sich in der Regel der Eindringwinkel der Zahnoberfläche bzw. der Kaufläche in die Tablette. Verschiedene Eindringwinkel können durch die Drehung des Stabes 20 um seine Längsachse LA nachgebildet werden.

Hierbei stellt sich das technische Problem, um die Drehbarkeit des Stabes 20 zu gewährleisten, dass die an dem Stab 20 angeordneten Gewichte 6 bzw. die Trägerplatte 7 von dem Stab 20 entkoppelt werden müssen. Damit die Gewichtskraft der Gewichte 6 über den Stab 20 auf die Probe eingeleitet werden kann, müssen die Gewichte allerdings fest mit der Achse verbunden sein bzw. muss die Trägerplatte 7, auf der die Gewichte aufliegen, mit dem Stab 20 verbunden sein. Das Problem hierbei ist, dass die Trägerplatte 7 nicht drehbar gelagert ist bzw. nicht drehbar gelagert werden kann, sodass durch eine feste Verbindung der Trägerplatte 7 mit dem Stab 20 eine Drehung des Stabes 20 um seine Längsachse LA verhindert werden würde.

Um sowohl die vertikale Bewegung des Stabes 20 als auch die Drehbarkeit des Stabes 20 um seine Längsachse LA zu gewährleisten, wird die Trägerplatte 7 über ein, vorzugsweise über zwei, Axialkugellager 19 mit dem Stab 20 verbunden. Dadurch kann die Gewichtskraft der Gewichte 6 auf den Stab 20 wirken, wobei gleichzeitig die Drehbarkeit des Stabes 20 gewährleistet ist.

Um eine vertikale spielfreie Führung des Stabes 20 zu gewährleisten, läuft der Stab 20 in einem, vorzugsweise in zwei, Linearkugellagern 21. Die Linearkugellager 21 sind in eine Hülse 22 eingepresst.

Die Hülse 22 wiederum ist in zwei Radialkugellagern 25 gelagert, um ein Drehen der Hülse 22 um die Längsachse LA des Stabes 20 zu gewährleisten. Durch Drehen der Hülse 22 wird auch der Stab 20 mitgedreht. Die Drehbewegung der Hülse 22 wird durch einen Schrittmotor 23 erreicht, der über eine Zahnradkombination 24 oder eine Zahnriemenkoppelung die Hülse antreibt.

Damit die Hülse 22 bei der Drehbewegung den Stab 20 mitnehmen kann, weist die Hülse 22 eine Schraube bzw. einen Stift 30 auf, welcher in ein Langloch 29 am Stab 20 eingreift. Der Stift bzw. die Schraube 30 kann so bei einer Drehbewegung der Hülse 22 den Stab 20 mitnehmen. Die Länge des Langloches 29 ist so ausgelegt, dass eine maximal notwendige Verfahrbarkeit des Stabes 20 in vertikaler Richtung gewährleistet ist.

Ferner ist ein in Fig. 5 nicht gezeigter Wegsensor vorgesehen, um die Relativposition des Stabes 20 bezogen auf die Trägereinrichtung 4 zu ermitteln. In einer Ausführungsform der Erfindung ist der Wegsensor als induktiver Wegsensor ausgebildet. Mit dem Wegsensor kann die Eindringtiefe des Belastungskörpers 5 in die Probe gemessen werden. Beim Eindringen des Belastungskörpers 5 in die Probe verändert sich (vorzugsweise bei feststehender Trägereinrichtung 4) der Abstand zwischen der Trägerplatte 7 und der Trägereinrichtung 4. Dieser Abstand wird mit dem Wegsensor gemessen. Bei einem induktiven Wegsensor wird ein metallischer Kern an einem Kunststoffstab befestigt, der mit der Trägerplatte 7 verbunden ist. Der Sensor selbst besteht aus einer Spule, in die der metallische Kern des Stabes eintaucht. Alternativ kann der metallische Kern an dem Kunststoffstab auch an der Trägereinrichtung 4 befestigt werden.

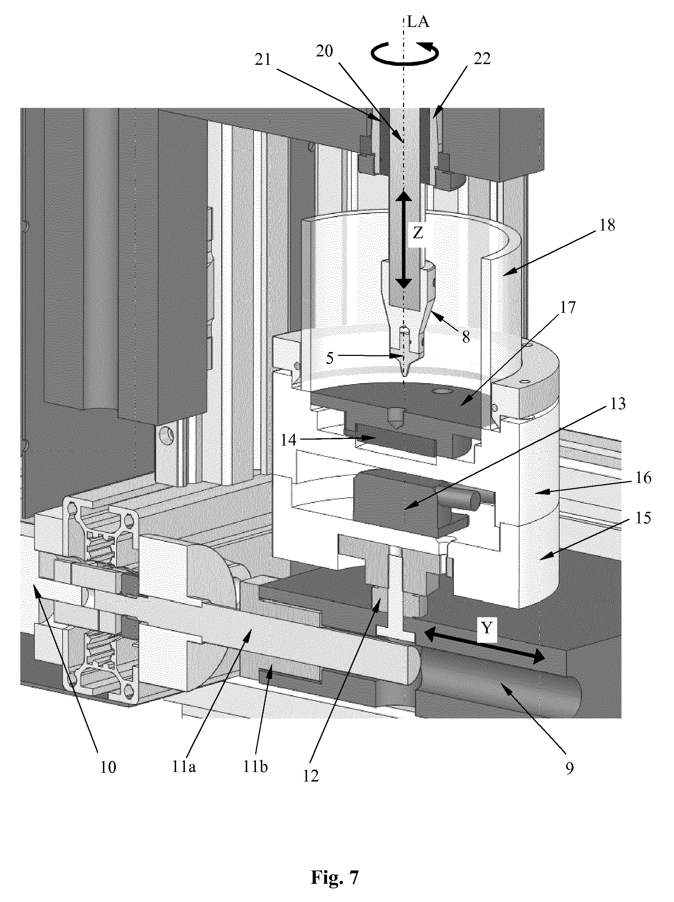

**Fig. 6** und **Fig. 7** zeigen eine Detailansicht der Probenkammer in einer Schnittansicht.

Die Probenkammer 28 ist hier mehrteilig aufgebaut und umfasst ein Unterteil 15, ein Oberteil 16 und einen an dem Oberteil 16 angeordneten Zylinder 18, welcher vorzugsweise aus Polymethylmethacrylat besteht.

Das Unterteil 15 der Probenkammer weist an der Oberseite eine Aussparung auf. Das Oberteil 16 der Probenkammer weist an der Unterseite eine Aussparung auf. Die beiden Aussparungen bilden zusammen einen im Wesentlichen wasserdichten Hohlraum, in welchem eine Umwälzpumpe 13 angeordnet ist.

Das Oberteil 16 der Probenkammer weist an der Oberseite ebenfalls eine Aussparung auf, in welcher eine Heiz-/Kühleinrichtung angeordnet ist. Die oberseitige Aussparung des Oberteils 16 der Probenkammer sowie die Heiz-/Kühleinrichtung 14 werden von einer vorzugsweise wärmeleitenden Platte abgedeckt, sodass die oberseitige Aussparung zusammen mit der wärmeleitenden Platte 17 ebenfalls einen im Wesentlichen wasserdichten Hohlraum bilden. Die Heiz-/Kühleinrichtung 14 ist mit der wärmeleitenden Platte verbunden. Die wärmeleitende Platte 17 bildet den Boden des Zylinders 18. Mit der Heiz-/Kühleinrichtung 14 und der damit verbundenen Platte 17 wird das in dem Zylinder vorhandene Wasser erwärmt.

Der Hohlraum zwischen dem Unterteil 15 und dem Oberteil 16 bzw. die Umwälzpumpe ist über zwei Kanäle mit dem Innenraum des Zylinders 18 verbunden, wobei in Fig. 7 nur ein Kanal sichtbar ist. Über einen Kanal wird Wasser in den Zylinder gepumpt, während über den anderen Kanal Wasser aus dem Zylinder entnommen wird. Mit der Umwälzpumpe 13 kann so eine homogene Temperaturverteilung des Wassers in dem Zylinder 18 erreicht bzw. sichergestellt werden. Die gesamte Probenkammer wird auf einen Kraftsensor 12 angeordnet, welcher mit dem Probentisch 9 verbunden ist, wie mit Bezug auf Fig. 3 und Fig. 4 erläutert.

Besonders gut erkennbar sind in Fig. 6 und Fig. 7 der Belastungskörper 5, welcher hier als rotationssymmetrischer Belastungskörper ausgestaltet ist. Selbstverständlich können auch nicht-rotationssymmetrische, etwa schräg abgekantete Belastungskörper 5 vorgesehen sein. Die Unterseite des Belastungskörpers 5 kann aber auch einer Kaufläche eines Zahnes nachempfunden sein.

Um einen Belastungstest durchzuführen, wird zunächst die Probe, etwa eine Tablette, in der Probenkammer 28 angeordnet, wobei die Probe in der Probenkammer befestigt werden kann, um ein Aufschwimmen im Wasser, welches sich gegebenenfalls in der Probenkammer befindet, zu verhindern.

Anschließend können Versuchsparameter der Belastungsmesseinrichtung festgelegt werden. Beispielsweise können die Verfahrwege des Stabes 20, der Probenkammer 28 bzw. des Probentisches 9, die Verfahrgeschwindigkeiten des Stabes 20, der Probenkammer 28 bzw. des Probentisches 9, der Drehwinkel des Stabes 20, die Winkelgeschwindigkeit des Stabes 20, die Anzahl der Hubbewegungen des Stabes entlang der Z-Achse festgelegt werden. Die Versuchsparameter können in einer Speichereinrichtung der vorstehend genannten Datenverarbeitungseinrichtung gespeichert werden, sodass sie für spätere Versuche aus der Speichereinrichtung abgerufen werden können.

Ferner können sogenannte Abbruchkriterien für einen Versuch festgelegt werden. Die Abbruchkriterien können beispielsweise eine maximale Eindringtiefe des Belastungskörpers 5 in die Probe, die maximale Kraft in Y- und/oder Z-Richtung, die maximale Anzahl von Hubbewegungen, und/oder den maximalen Drehwinkel des Stabes 20 umfassen. Auch die Abbruchkriterien können in der Speichereinrichtung abgespeichert werden, sodass auch diese für spätere Versuche aus der Speichereinrichtung ausgelesen werden können.

Sobald die Versuchsparameter und gegebenenfalls die Abbruchkriterien festgelegt wurden bzw. aus der Speichereinrichtung ausgelesen wurden, kann der Versuch gestartet bzw. durchgeführt werden. Während des Versuchsablaufes werden verschiedene Messwerte aufgezeichnet, insbesondere die auf die Probe wirkenden Kräfte in X- und/oder Y- und/oder Z-Richtung und die Eindringtiefe des Belastungskörpers in die Probe. Aus den aufgezeichneten Messwerten kann ein Kraft-/Zeitdiagramm in X-/Y-/Z-Richtung erstellt und gegebenenfalls in der Speichereinrichtung abgelegt werden. Die aufgenommenen Messwerte bzw. die daraus erzeugten Kraft-/Zeitdiagramme können an einer Anzeigeeinrichtung ausgegeben werden.

Ferner kann die Probenkammer mit einem Fluid, etwa Wasser, gefüllt werden. Dem Wasser können weitere Zusatzstoffe beigegeben werden, um etwa den pH-Wert des Wassers zu ändern. Ferner kann auch die Temperatur des Fluids als Versuchsparameter festgelegt und gegebenenfalls in der Speichereinrichtung abgespeichert werden.

Bei dem Festlegen der Versuchsparameter kann auch festgelegt werden, in welcher Reihenfolge welche Bewegungen durchgeführt werden sollen.

In einer bevorzugten Ausführungsform der Erfindung kann eine maximale Belastungskraft von 350 N vorgesehen sein, welche durch Gewichte von 1 kg und 2 kg erreicht werden. Das maximale Volumen des Zylinders kann etwa 200 ml betragen. Im Zylinder 18 kann ein Temperaturfühler vorgesehen sein, um die Heiz-/Kühleinrichtung 14 zu steuern.

### Bezugszeichenliste

- 1: erste Antriebseinheit (erster Servomotor) zum Bewegen des Belastungskörpers entlang der Z-Achse
- 2a, 2b: erste Vorschubeinrichtung (erstes Kugelgewindetriebe), welche mit der ersten Antriebseinheit gekoppelt ist
- 2a: Kugelgewindespindel des ersten Kugelgewindetriebes
- 2b: Kugelgewindemutter des ersten Kugelgewindetriebes
- 4: Trägereinrichtung, welche mit der Kugelgewindemutter des ersten Kugelgewindetriebes gekoppelt ist
- 5: Belastungskörper
- 6: Gewichte
- 7: Trägerplatte zur Aufnahme der Gewichte
- 8: Verbindungselement zur Aufnahme des Belastungskörpers
- 9: Probentisch
- 10: zweite Antriebseinheit (zweiter Servomotor) zum Bewegen des Probentisches entlang der Y-Achse
- 11a, 11b: zweite Vorschubeinrichtung (zweites Kugelgewindetriebe), welche mit der zweiten Antriebseinheit gekoppelt ist
- 11a: Kugelgewindespindel des zweiten Kugelgewindetriebes
- 11b: Kugelgewindemutter des zweiten Kugelgewindetriebes
- 12: Kraftsensor
- 13: Umwälzpumpe
- 14: Heiz-/Kühleinrichtung
- 15: Unterteil der Probenkammer
- 16: Oberteil der Probenkammer
- 17: Wärme leitende Platte (vorzugsweise metallische Platte)
- 18: Zylinder (vorzugsweise aus Polymethylmethacrylat)
- 19: Axiallager, vorzugsweise Axialkugellager
- 20: Stab, vorzugsweise zylinderförmig und in Z-Richtung bewegbar und um seine Längsachse drehbar
- 21: Linearkugellager
- 22: Hülse um die Linearkugellager
- 23: dritte Antriebseinheit (Schrittmotor), welcher mit der Hülse gekoppelt ist
- 24: Zahnradkombination zum Koppeln des Schrittmotors mit der Hülse
- 25: Radiallager, vorzugsweise Radialkugellager
- 26: Tür / Klappe der Belastungseinrichtung
- 27: Füße der Belastungseinrichtung
- 28: Probenkammer
- 29: Langloch am Stab (parallel zur Längsachse des Stabes)
- 30: Stift (Sicherungsschraube) an der Hülse, welcher in das Langloch eingreift
- LA: Längsachse des Stabes
- Y: erste Achse
- Z: zweite Achse

## Patentansprüche

1. Belastungsmesseinrichtung, insbesondere zum Messen physikalischer Parameter einer Probe bei einer mechanischen Krafteinwirkung auf die Probe, aufweisend
- eine Probenkammer (28) zur Aufnahme der Probe, wobei die Probenkammer entlang einer ersten Achse (Y) verfahrbar ist, und
- einen Stab (20), welcher entlang einer zweiten Achse (Z) relativ zur Probenkammer (28) verfahrbar ist, wobei die zweite Achse (Z) im Wesentlichen senkrecht zur ersten Achse (Y) steht, und wobei an dem der Probenkammer (28) zugewandten Ende des Stabes (20) ein Belastungskörper (5) angeordnet ist,
**dadurch gekennzeichnet, dass** der Stab (20) drehbar um seine Längsachse (LA) gelagert ist.

2. Belastungsmesseinrichtung nach dem vorhergehenden Anspruch, wobei der Stab (20), über zumindest ein Axiallager (19), mit einer Trägerplatte (7) zur Aufnahme von Gewichten (6) gekoppelt ist.

3. Belastungsmesseinrichtung nach Anspruch 2, wobei zum Verfahren des Stabes (20) entlang der zweiten Achse (Z) eine erste Antriebseinheit (1) vorgesehen ist, welche, vorzugsweise über eine erste Vorschubeinrichtung (2a, 2b), mit einer entlang der zweiten Achse (Z) verfahrbaren Trägereinrichtung (4) gekoppelt ist, wobei die Trägereinrichtung (4) derart relativ zur Trägerplatte (7) angeordnet ist, dass ein Verfahren der Trägereinrichtung (4) ein Verfahren der Trägerplatte (7) entlang der zweiten Achse (Z) bewirkt.

4. Belastungsmesseinrichtung nach einem der vorhergehenden Ansprüche, weiter aufweisend eine dritte Antriebseinheit (23), welche, vorzugsweise über Koppelmittel, mit dem Stab (20) gekoppelt ist, um den Stab um dessen Längsachse (LA) zu drehen.

5. Belastungsmesseinrichtung nach Anspruch 4, wobei die Koppelmittel eine Zahnradkombination (24) oder Zahnriemenkoppelung und eine Hülse (22) umfassen, wobei die Zahnradkombination (24) oder die Zahnriemenkoppelung mit der dritten Antriebseinheit (23) und mit der Hülse (22) gekoppelt ist, wobei die Hülse (22) den Stab (20) zumindest teilweise umgibt, und wobei die Hülse (22) an der Innenwandung einen Stift (30) aufweist, welcher in ein parallel zur Längsache (LA) verlaufendes Langloch (29) am Stab (20) eingreift.

6. Belastungsmesseinrichtung nach Anspruch 5, wobei in der Hülse (22) zumindest ein Linearlager (21) angeordnet ist, in welchem der Stab (20) im Wesentlichen spielfrei entlang seiner Längsachse (LA) geführt ist.

7. Belastungsmesseinrichtung nach einem der Ansprüche 3 bis 6, wobei die dritte Antriebseinheit (23) und die Koppelmittel an der Trägereinrichtung (4) angeordnet sind, wobei die Trägereinrichtung (4) einen Durchbruch aufweist, in welchem die Hülse (22) in zumindest einem Radiallager (25) drehbar gelagert ist.

8. Belastungsmesseinrichtung nach einem der vorhergehenden Ansprüche, wobei die Probenkammer (28) auf einem Probentisch (9) angeordnet ist, welcher mit einer zweiten Antriebseinheit (10), vorzugsweise über eine zweite Vorschubeinrichtung (11a, 11b), gekoppelt ist.

9. Belastungsmesseinrichtung nach Anspruch 8, wobei zwischen dem Probentisch (9) und der Probenkammer (28) ein Kraftsensor (12), vorzugsweise ein Mehrkomponenten-Kraftsensor, angeordnet ist und/oder wobei ein Wegsensor, vorzugsweise ein induktiver Wegsensor zum Messen des vertikalen Verfahrweges des Stabes (20) vorgesehen ist.

10. Belastungsmesseinrichtung nach einem der vorhergehenden Ansprüche, wobei in der Probenkammer (28) eine Heiz-/Kühleinrichtung (14), eine Umwälzpumpe (13) und eine Platte (17) zur Aufnahme einer Probenhalterung angeordnet sind.

11. Belastungsmesseinrichtung nach Anspruch 10, wobei die Probenkammer (28) mehrteilig ausgestaltet ist, wobei ein Unterteil (15) und ein Oberteil (16) der Probenkammer zusammen eine erste Kammer bilden, in der die Umwälzpumpe (13) angeordnet ist, wobei die Platte (17) an der Oberseite des Oberteils (16) angeordnet ist und mit dem Oberteil (16) eine fluiddichte zweite Kammer bildet, in der die Heiz-/Kühleinrichtung (14) angeordnet ist, wobei die Heiz-/Kühleinrichtung (14) vorzugsweise thermisch mit der Platte (17) gekoppelt ist, wobei an der Oberseite des Oberteils (16) ein offener Zylinder (18) angeordnet ist, dessen Grundfläche von der Platte (17) fluiddicht verschlossen wird, und wobei die erste Kammer über zumindest einen Kanal mit dem Innenraum des Zylinders verbunden ist.

12. Belastungsmesseinrichtung nach einem der vorhergehenden Ansprüche, wobei die erste Vorschubeinrichtung (2a, 2b) und / oder die zweite Vorschubeinrichtung (11a, 11b) einen Kugelgewindetrieb mit einer Kugelgewindespindel (2a, 11a) und einer Kugelgewindemutter (2b, 11b) umfassen, und / oder wobei die erste Antriebseinheit (1) und / oder die zweite Antriebseinheit (10) einen Servomotor umfassen, und / oder wobei die dritte Antriebseinheit (23) einen Schrittmotor umfasst.

13. Verfahren zum Messen physikalischer Parameter einer Probe bei einer mechanischen Krafteinwirkung auf die Probe, mit einer Belastungsmesseinrichtung nach einem der vorhergehenden Ansprüche, wobei das Verfahren zumindest umfasst:
- Anordnen der Probe in einer Probenkammer (28),
- Festlegen von Versuchsparametern der Belastungsmesseinrichtung, wobei die Versuchsparameter zumindest eines aus Drehwinkel des Stabes (20), Winkelgeschwindigkeit des Stabes (20), und eine Kombination hiervon umfassen,
- Festlegen von Abbruchkriterien, wobei die Abbruchkriterien zumindest eines aus maximaler Eindringtiefe des Belastungskörpers (5) in die Probe, maximaler Kraft in Y- und/oder Z-Richtung, maximaler Anzahl von Hubbewegungen, maximaler Drehwinkel des Stabes (20), und eine Kombination hiervon umfassen,
- Aufzeichnen von Messwerten, insbesondere Kraft/Zeitdiagramm in Y- und/oder Z-Richtung und Eindringtiefe.

14. Verfahren nach dem vorhergehenden Anspruch, wobei die Versuchsparameter weiter umfassen: Verfahrweg des Stabes (20), Verfahrweg der Probenkammer (28), Verfahrgeschwindigkeit des Stabes (20), Verfahrgeschwindigkeit der Probenkammer (28), Anzahl von Hubbewegungen des Stabes entlang der zweiten Achse (Z).

15. Verfahren nach Anspruch 13 oder 14, wobei das Verfahren ferner umfasst:
- Füllen der Probenkammer mit einem Fluid, und
- Festlegen der Temperatur des Fluids als Versuchsparameter.

## Claims

1. A load measuring device, in particular for measuring physical parameters of a sample when said sample is exposed to the application of a mechanical force, said load measuring device comprising:
- a sample chamber (28) for receiving said sample, the sample chamber being displaceable along a first axis (Y), and
- a rod (20) that is displaceable with respect to the sample chamber (28) along a second axis (Z), said second axis (Z) being essentially perpendicular to the first axis (Y), with a load application body (5) being arranged at the end of the rod (20) facing towards the sample chamber (28),
**characterised in that** the rod (20) supported in such a manner that it is rotatable about its longitudinal axis (LA).

2. The load measuring device as claimed in the preceding claim, wherein the rod (20) is coupled, via at least one axial bearing (19), to a support plate (7) to be loaded with weights (6).

3. The load measuring device as claimed in claim 2, wherein provision is made for the displacement of the rod (20) along the second axis (Z) to be carried out by a first drive unit (1) which is coupled to a carrier device (4) displaceable along the second axis (Z), preferably via a first advance device (2a, 2b), said carrier device (4) being arranged in such a manner relative to said support plate (7) that displacement of the carrier device (4) will cause the support plate (7) to be displaced along the second axis (Z).

4. The load measuring device as claimed in any of the preceding claims, further comprising a third drive unit (23) which is coupled to the rod (20), preferably via coupling means, in order to rotate the rod about its longitudinal axis (LA).

5. The load measuring device as claimed in claim 4, wherein said coupling means comprise a combination of gears (24) or toothed belt coupling and a sleeve (22), said combination of gears (24) or toothed belt coupling being coupled to the third drive unit (23) and to the sleeve (22), said sleeve (22) surrounding at least partially the rod (20), and said sleeve (22) having a dowel pin (30) arranged on the inner wall thereof which engages with an elongated hole (29) that is formed in the rod (20) and extends parallel with the longitudinal axis (LA) thereof.

6. The load measuring device as claimed in claim 5, wherein the sleeve (22) has at least one linear bearing (21) arranged therein in which the rod (20) is guided along its longitudinal axis (LA) in an essentially free-of-play manner.

7. The load measuring device as claimed in any one of claims 3 to 6, wherein the third drive unit (23) and the coupling means are arranged on the carrier device (4), said carrier device (4) having an aperture in which the sleeve (22) is supported in a manner so as to be rotatable in at least one radial bearing (25).

8. The load measuring device as claimed in any of the preceding claims, wherein the sample chamber (28) is arranged on a sample table (9) which is coupled to a second drive unit (10), preferably via a second advance device (11 a, 11 b).

9. The load measuring device as claimed in claim 8, wherein a force sensor (12), preferably a multi-component force sensor, is arranged between the sample table (9) and the sample chamber (28), and/or wherein a displacement sensor, preferably an inductive displacement sensor, is provided for measuring the vertical displacement of the rod (20).

10. The load measuring device as claimed in any of the preceding claims, wherein the sample chamber (28) has a heating/cooling device (14), a circulating pump (13), and a plate (17) for accomodating a sample holder arranged therein.

11. The load measuring device as claimed in claim 10, wherein the sample chamber (28) is configured in several parts, a lower part (15) and an upper part (16) of the sample chamber cooperating to form a first chamber (16) in which the circulating pump (13) is arranged, the plate (17) being arranged on the top surface of the upper part (16) and cooperating with the upper part (16) to form a fluid-tight, second chamber in which the heating/cooling device (14) is arranged, said heating/cooling device (14) being preferably thermally coupled to the plate (17), the top surface of the upper part (16) having an open cylinder (18) arranged thereon the base of which is closed in a fluid-tight manner by the plate (17), said first chamber being connected with the interior of said cylinder via at least one channel.

12. The load measuring device as claimed in any of the preceding claims, wherein the first advance device (2a, 2b) and/or the second advance device (11a, 11b) include a ball screw having a ball screw spindle (2a, 11 a) und a ball screw nut (2b, 11 b), and/or wherein the first drive unit (1) and/or the second drive unit (10) include a servomotor, and/or wherein the third drive unit (23) includes a stepper motor.

13. A method of measuring physical parameters of a sample when said sample is exposed to the application of a mechanical force, said method using a load measuring device as claimed in any of the preceding claims, wherein said method comprises at least:
- disposing the sample in a sample chamber (28),
- establishing test parameters of the load measuring device, said test parameters including at least one of a rotation angle of the rod (20), an angular velocity of the rod (20), and any combination thereof,
- establishing termination criteria, said termination criteria including at least one of a maximum penetration depth of the load application body (5) into the sample, a maximum force exerted in the y and/or z direction(s), a maximum number of stroke movements, a maximum rotation angle of the rod (20), and any combination thereof,
- recording of measured values, in particular force-time diagram in the x and/or y directions and penetration depth.

14. The method as claimed in any of the preceding claims, wherein the test parameters further include the travel of the rod (20), the travel of the sample chamber (28), the travelling speed of the rod (20), the travelling speed of the sample chamber (28), the number of stroke movements of the rod along the second axis (Z).

15. The method as claimed in claim 13 or 14, wherein the method further includes:
- filling of the sample chamber with a fluid, and
- establishing the temperature of the fluid as a test parameter.

## Revendications

1. Dispositif de mesure de charge, en particulier destiné à mesurer des paramètres physiques d'un échantillon lors d'une force exercée sur l'échantillon, lequel dispositif présente :
- une chambre d'échantillon (28) destinée à recevoir l'échantillon, la chambre d'échantillon pouvant être déplacée le long d'un premier axe (y) et
- une tige (20) qui peut être déplacée par rapport à la chambre d'échantillon (28) le long d'un deuxième axe (z), le deuxième axe (z) étant essentiellement perpendiculaire au premier axe (y) et un corps de charge (5) étant disposé à l'extrémité de la tige (20) tournée vers la chambre d'échantillon (28),
**caractérisé en ce que** la tige (20) est logée de manière à pouvoir tourner autour de son axe longitudinal (LA).

2. Dispositif de mesure de charge selon la revendication précédente, dans lequel la tige (20) est couplée à une plaque support (7) par le biais d'au moins un roulement axial (19), et ce en vue de recevoir des poids (6).

3. Dispositif de mesure de charge selon la revendication 2, dans lequel en vue de déplacer la tige (20) le long du deuxième axe (z) est prévue une première unité d'entraînement (1) qui est couplée, de préférence par le biais d'un premier dispositif d'avance (2a, 2b), à un dispositif porteur (4) qui peut être déplacé le long du deuxième axe (z), le dispositif porteur (4) étant disposé de telle sorte par rapport à la plaque support (7) qu'un déplacement du dispositif porteur (4) provoque nécessairement un déplacement de la plaque support (7) le long du deuxième axe (z).

4. Dispositif de mesure de charge selon l'une quelconque des revendications précédentes, lequel présente en outre une troisième unité d'entraînement (23) qui est couplée, de préférence par le biais de moyens de couplage, à la tige (20) afin de faire tourner ladite tige autour de son axe longitudinal (LA).

5. Dispositif de mesure de charge selon la revendication 4, dans lequel les moyens de couplage comprennent une combinaison de roues dentées (24) ou un couplage à courroie crantée et une douille (22), la combinaison de roues dentées (24) ou le couplage à courroie crantée étant couplé(e) à la troisième unité d'entraînement (23) et à la douille (22), la douille (22) entourant au moins partiellement la tige (20) et la douille (22) présentant, sur la paroi intérieure, un goujon (30) qui entre dans un trou oblong (29) ménagé dans la tige (20) et s'étendant parallèlement à l'axe longitudinal (LA).

6. Dispositif de mesure de charge selon la revendication 5, dans lequel dans la douille (22) est disposé au moins un roulement linéaire (21) dans lequel la tige (20) est conduite essentiellement sans jeu le long de son axe longitudinal (LA).

7. Dispositif de mesure de charge selon l'une quelconque des revendications 3 à 6 dans lequel la troisième unité d'entraînement (23) et les moyens de couplage sont disposés sur le dispositif porteur (4), le dispositif porteur (4) présentant une ouverture dans laquelle la douille (22) est logée dans au moins un roulement radial (25) de manière à pouvoir tourner.

8. Dispositif de mesure de charge selon l'une quelconque des revendications précédentes, dans lequel la chambre d'échantillon (28) est disposée sur une table d'échantillon (9) qui est couplée à une deuxième unité d'entraînement (10), de préférence par le biais d'un deuxième dispositif d'avance (11a, 11b).

9. Dispositif de mesure de charge selon la revendication 8, dans lequel un capteur de force (12), de préférence un capteur de force à plusieurs constituants, est disposé entre la table d'échantillon (9) et la chambre d'échantillon (28) et/ou dans lequel un capteur de course, de préférence un capteur de course inductif, est prévu en vue de mesurer la distance de déplacement de la tige (20).

10. Dispositif de mesure de charge selon l'une quelconque des revendications précédentes, dans lequel un dispositif chauffant/refroidissant (14), une pompe de circulation (13) et une plaque (17) destinée à recevoir un porte-échantillon sont disposés dans la chambre d'échantillon (28).

11. Dispositif de mesure de charge selon la revendication 10, dans lequel la chambre d'échantillon (28) est réalisée en plusieurs parties, une partie inférieure (15) et une partie supérieure (16) de la chambre d'échantillon formant ensemble une première chambre dans laquelle est disposée la pompe de circulation (13), la plaque (17) étant disposée sur la face supérieure de la partie supérieure (16) et formant, avec la partie supérieure (16), une deuxième chambre étanche aux fluides dans laquelle est disposé le dispositif chauffant/refroidissant (14), le dispositif chauffant/refroidissant (14) étant couplé, de préférence de manière thermique, à la plaque (17), sur la face supérieure de la partie supérieure (16) étant disposé un cylindre ouvert (18) dont la surface de base est fermée par la plaque (17) de manière hermétique aux fluides et la première chambre étant reliée à l'espace intérieur dudit cylindre par au moins un canal.

12. Dispositif de mesure de charge selon l'une quelconque des revendications précédentes, dans lequel le premier dispositif d'avance (2a, 2b) et/ou le deuxième dispositif d'avance (11 a, 11 b) comprennent une vis à billes pourvue d'une tige filetée (2a, 11a) et d'un écrou (2b, 11b), et/ou la première unité d'entraînement (1) et/ou la deuxième unité d'entraînement (10) comprennent un servomoteur, et/ou la troisième unité d'entraînement (23) comprend un moteur pas à pas.

13. Procédé destiné à mesurer des paramètres physiques d'un échantillon lors d'une action mécanique exercée par une force sur l'échantillon, et ce à l'aide d'un dispositif de mesure de charge selon l'une quelconque des revendications précédentes, le procédé comprenant au moins les étapes suivantes :
- la disposition de l'échantillon dans une chambre d'échantillon (28),
- la détermination de paramètres d'essai du dispositif de mesure de charge, les paramètres d'essai comprenant au moins une des données représentant l'angle de rotation de la tige (20), la vitesse angulaire de la tige (20), et une combinaison de celles-ci,
- la détermination de critères d'arrêt, les critères d'arrêt comprenant au moins une des données représentant la profondeur de pénétration maximale du corps de charge (5) dans l'échantillon, la force maximale en direction y et/ou en direction z, le nombre maximal de mouvements verticaux, l'angle de rotation maximal de la tige (20), et une combinaison de celles-ci,
- l'enregistrement de valeurs de mesure, en particulier un diagramme force-temps en direction y et/ou z et la profondeur de pénétration.

14. Procédé selon la revendication précédente, lors duquel les paramètres d'essai comprennent : la distance de déplacement de la tige (20), la distance de déplacement de la chambre d'échantillon (28), la vitesse de déplacement de la tige (20), la vitesse de déplacement de la chambre d'échantillon (28), le nombre de mouvements verticaux de la tige le long du deuxième axe (z).

15. Procédé selon la revendication 13 ou 14, ledit procédé comprenant en outre :
- le remplissage de la chambre d'échantillon avec un fluide et
- la détermination de la température du fluide en tant que paramètre d'essai.
